# EUROPEAN PATENT APPLICATION

(11) **EP 3 251 637 A1**
(43) Date of publication of application: **06.12.2017**
(21) Application number: 16743383.8
(22) Date of filing: 27.01.2016
(51) Int. Cl.: A61F 7/03

(54) **HEAT GENERATION TOOL**

(30) Priority: 29.01.2015 JP 2015016121
(71) Applicant: Kobayashi Pharmaceutical Co., Ltd., Chuo-ku Osaka-shi, Osaka 541-0045 (JP)
(72) Inventor: NISHIOKA, Daisuke, Osaka-shi, Osaka 532-0033 (JP)
(74) Representative: Vossius & Partner Patentanwälte Rechtsanwälte mbB
(86) International application number: PCT/JP2016/052234
(87) International publication number: WO 2016/121780

(57) **Abstract**

This invention provides a heating tool that is capable of significantly suppressing a reduction of an active ingredient contained in an adhesive layer. More specifically, the invention provides a heating tool comprising an exothermic composition containing an oxidation accelerator, an oxidizable metal powder, and water; an active ingredient-containing adhesive layer; and a vapor deposition layer, the exothermic composition being housed in an air-permeable container bag, the vapor deposition layer being disposed between the container bag and the adhesive layer.

## Description

### Technical Field

The present invention relates to a heating tool.

### Background Art

Various heretofore known components exhibit a characteristic effect according to their type, such as pain relief, inflammation inhibition, blood circulation promotion, relaxation, and cool sensation. As means for obtaining such a characteristic effect attributable to active ingredients, there have been numerous reports for patches, cataplasms, ointments, and aerosols, each containing the component, as well as devices that diffuse the component with electricity or fire.

Heating tools, such as disposable body warmers, have been used as a warming tool for the body or as a thermal therapy device. In particular, disposable body warmers have been used frequently because they have excellent properties, such as portability, safety, and convenience, and also because they are inexpensive. General-purpose disposable body warmers use an exothermic composition that generates heat in the presence of air, and a heat-retention effect is produced through this heat-generating mechanism.

A conventional technique of using a heating tool or other disposable body warmers in combination with a fragrance has also been reported. For example, Patent Literature (PTL) 1 discloses adding a fragrance to the surface of a hitherto-known disposable body warmer, and promoting the volatilization and diffusion of the fragrance by utilizing the heat-generating mechanism of the disposable body warmer. In this manner, as reported in PTL 1, safety and convenience are improved at the time of diffusion of the fragrance, its portability is improved, and an immediate use is made possible without impairing the effect.

Even in the current situation above, from the viewpoint of obtaining furthermore improved effects, it is still important to provide a technique by which a useful effect attributable to the components mentioned above are more satisfactorily exerted.

### Citation List

### Patent Literature

PTL 1: JP2001-218816A

### Summary of Invention

### Technical Problem

Accordingly, an object of the present invention is to provide a means for more satisfactorily exerting a useful effect attributable to an active ingredient. In particular, the present inventor found that a useful effect attributable to an active ingredient is likely to be significantly impaired when provided with a heat-generating mechanism, in which heat is generated in the presence of air, found in general-purpose disposable body warmers; the inventor focused on this point.

Accordingly, an object of the present invention is to provide a heating tool that is capable of more effectively exerting a useful effect attributable to an active ingredient while using a heat-generating mechanism. Another object of the present invention is to provide a method for more effectively obtaining a useful effect attributable to an active ingredient while using the heat-generating mechanism.

### Solution to Problem

The present inventor conducted extensive research to solve the above problem, and found the following: when an active ingredient-containing adhesive layer is disposed via a vapor deposition layer on an air-permeable container bag housing an exothermic composition containing an oxidation accelerator, an oxidizable metal powder, and water, a reduction of the active ingredient contained in the adhesive layer is significantly suppressed, even after long-term storage. The present invention has been accomplished as a result of further study based on this finding. More specifically, the present invention provides the following invention.

Item 1. A heating tool comprising:
an exothermic composition containing an oxidation accelerator, an oxidizable metal powder, and water;
an active ingredient-containing adhesive layer; and
a vapor deposition layer,
the exothermic composition being housed in an air-permeable container bag, the vapor deposition layer being disposed between the container bag and the adhesive layer.

Item 2. The heating tool according to Item 1, for direct application to the skin via the adhesive layer.

Item 3. The heating tool according to Item 1 or 2, wherein the oxidation accelerator is carbon black.

Item 4. The heating tool according to any one of Items 1 to 3, wherein the exothermic composition further contains a water-soluble salt and/or a water-retaining agent.

Item 5. The heating tool according to any one of Items 1 to 4, wherein the exothermic composition contains the oxidation accelerator in an amount of 1 to 30 wt%.

Item 6. The heating tool according to any one of Items 1 to 5, wherein the adhesive layer contains the active ingredient in an amount of 0.00001 to 85 wt%.

Item 7. A method for suppressing a reduction of an active ingredient in a heating tool comprising an active ingredient-containing adhesive layer, the method comprising disposing a vapor deposition layer between the active ingredient-containing adhesive layer and a container bag housing an exothermic composition containing an oxidation accelerator, an oxidizable metal powder, and water.

### Advantageous Effects of Invention

According to the present invention, while the heat-generating mechanism is used, a reduction of an active ingredient contained in the adhesive layer is significantly suppressed; this makes it possible to more effectively exert the useful effect attributable to the active ingredient.

### Brief Description of Drawing

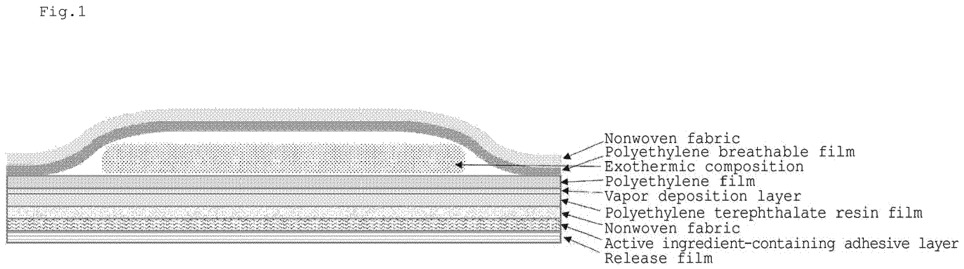

Fig. 1 is a model drawing of a heating tool according to the present invention. In Fig. 1, the exothermic composition is housed in a polyethylene container bag to which a nonwoven fabric is laminated, and a vapor deposition layer and an adhesive layer are laminated.

### Description of Embodiments

The heating tool of the present invention is characterized by comprising:
an exothermic composition containing an oxidation accelerator, an oxidizable metal powder, and water;
an active ingredient-containing adhesive layer; and
a vapor deposition layer.
The exothermic composition is housed in an air-permeable container bag, and the vapor deposition layer is disposed between the container bag and the adhesive layer. The following describes the heating tool of the present invention.

### Exothermic Composition

As used in known general-purpose disposable body warmers, an exothermic composition generates heat in the presence of air, and contains an oxidation accelerator, an oxidizable metal powder, and water.

The oxidation accelerator contained in the exothermic composition is used for the purpose of further promoting, by air intake, the supply of oxygen to the exothermic composition, in particular, to the oxidizable metal powder. The oxidation accelerator is not limited as long as oxygen is supplied. Examples of the oxidation accelerator include carbon black, graphite, activated carbon, coal, charcoal, bamboo charcoal, acetylene black, and charcoal made from waste coffee grounds. Of these, carbon black, activated carbon, bamboo charcoal, charcoal, and charcoal made from waste coffee grounds are preferable, and carbon black is more preferable. These may be used singly, or in a combination of two or more.

The oxidation accelerator is preferably, but not limited to, powdery, granular, fibrous, or the like as long as the desired effects are obtained, from the standpoint of comfort when the heating tool is attached to the body, the oxygen supply efficiency, and the like. These may be used singly, or in a combination of two or more. The oxidation accelerator has an average particle diameter of, for example, 0.001 to 1000 µm, preferably 0.005 to 500 µm, and more preferably 0.01 to 200 µm. The average particle diameter of the oxidation accelerator may be measured, for example, by using a standard sieve defined in JIS Z8801-01.

It is sufficient if the heating tool of the present invention generates heat to a temperature suitable for application to the skin. The temperature is, for example, about 32 to 85°C, and more preferably about 34 to 70°C (as measured based on JIS S4100 (2007)). To allow the heating tool to further efficiently generate heat to a more preferable temperature, the oxidation accelerator preferably has electrical conductivity. Oxidation accelerators having or not having electrical conductivity are known. Examples of known oxidation accelerators having electrical conductivity equal to or higher than a certain level include, but are not limited to, carbon black, graphite, activated carbon, and the like.

In the present invention, the exothermic temperature of the heating tool is measured according to JIS S4100 (2007). More specifically, a predetermined underlay material and a covering material are laid on a warming portion defined in JIS S4100 (2007). The warming portion is heated to 30°C, and held within one degree of that temperature. The heating tool left for 2 hours or more in an atmosphere having the same temperature as the ambient temperature is caused to generate heat based on the method of use, and the temperature is then measured in accordance with a predetermined method.

The amount of the oxidation accelerator is not limited as long as the desired effects are obtained. The proportion of the oxidation accelerator in the exothermic composition is, for example, 1 to 30 wt%, preferably 3 to 25 wt%, and more preferably 5 to 23 wt%.

The amount of the oxidation accelerator, relative to the oxidizable metal powder described later, is also not limited as long as the desired effects are obtained. The amount of the oxidation accelerator is, for example, 2 to 60 parts by weight, preferably 5 to 50 parts by weight, and more preferably 10 to 40 parts by weight, per 100 parts by weight of the oxidizable metal powder.

The oxidizable metal powder contained in the exothermic composition is not limited as long as it is a metallic powder that generates heat when being oxidized. Examples thereof include iron powder, zinc powder, aluminum powder, magnesium powder, and copper powder. A preferable example is iron powder. Examples of the iron powder include reduced iron powder, cast-iron powder, atomized iron powder, and electrolytic-iron powder. These may be used singly, or in a combination of two or more.

The oxidizable metal powder may be powdery, granular, or fibrous. These may be used singly, or in a combination of two or more.

The average particle diameter of the oxidizable metal powder is not limited as long as the desired effects are obtained, and is, for example, 0.01 to 1000 µm, preferably 0.1 to 500 µm, and more preferably 0.5 to 300 µm, from the standpoint of comfort when the heating tool is attached to the body, the heat generation efficiency, and the like. The average particle diameter of oxidizable metal powder may be measured, for example, by using a standard sieve defined in ZIS Z8801-1.

The amount of oxidizable metal powder is not limited as long as the desired effects are obtained. The proportion of the oxidizable metal powder in the exothermic composition is, for example, 20 to 80 wt%, preferably 25 to 70 wt%, and more preferably 30 to 60 wt%.

Usable water includes distilled water, tap water, ion exchange water, pure water, ultrapure water, industrial water, and the like.

The amount of water is also not limited as long as the desired effects are obtained. The proportion of water in the exothermic composition is, for example, 5 to 50 wt%, preferably 10 to 40 wt%, and more preferably 15 to 35 wt%.

In addition to the components described above, the exothermic composition may further optionally contain at least one member selected from the group consisting of water-soluble salts and water-retaining agents.

A water-soluble salt is incorporated in the exothermic composition to promote the oxidation of the oxidizable metal powder. The water-soluble salt is not limited as long as the desired effects are obtained. Preferable examples of the water-soluble salt include chloride salts and sulfide salts of alkali metals such as sodium and potassium; chloride salts and sulfide salts of alkaline earth metals such as calcium and magnesium; and chloride salts and sulfide salts of metals such as iron, copper, aluminum, zinc, nickel, silver, and barium. More preferable examples thereof include potassium chloride, sodium chloride, and the like. These may be used singly, or in a combination of two or more.

The amount of water-soluble salt is also not limited as long as the desired effects are obtained. The proportion of the water-soluble salt in the exothermic composition is, for example, 0.1 to 10 wt%, preferably 0.5 to 7 wt%, and more preferably 1 to 5 wt%.

The water-retaining agent, which has the function of retaining water, is not limited as long as it has the function and can produce desired effects. Examples thereof include porous substances, water-absorbing resins, and the like. Examples of the water-retaining agent include natural and synthetic inorganic substances such as vermiculite, perlite, calcium silicate, magnesium silicate, kaolin, talc, smectite, mica, bentonite, calcium carbonate, silica gel, alumina, zeolite, silicon dioxide, and diatomaceous earth; and natural and synthetic organic substances such as pulp, wood flour (sawdust), cotton, polyacrylate-based resins, polysulfonate-based resins, maleic anhydride salt-based resins, polyacrylamide-based resins, polyvinyl alcohol-based resins, polyethylene oxide-based resins, polyaspartate-based resins, polyglutamate-based resins, polyalginate-based resins, starches, and cellulose. Preferable examples thereof include vermiculite, wood flour, pulp, and polyacrylate-based resins, and more preferable examples thereof include vermiculite and polyacrylate-based resins. These may be used singly, or in a combination of two or more.

The average particle diameter of the water-retaining agent is not limited as long as the desired effects are obtained, and is, for example, 0.1 to 3000 µm, preferably 0.5 to 1000 µm, and more preferably 1 to 500 µm. The average particle diameter of the water-retaining agent is also measured, for example, by using a standard sieve defined in JIS Z8801-01, as in the measurement of the oxidation accelerator.

The amount of the water-retaining agent is also not limited as long as the desired effects are obtained. The proportion of the water-retaining agent in the exothermic composition is, for example, 1 to 20 wt%, preferably 3 to 15 wt%, and more preferably 5 to 10 wt%.

In these water-retaining agents, in particular, water-retaining agents having a porous structure, such as vermiculite, can serve not only as a water-retaining agent, but also as an air passageway.

In addition to the components mentioned above, the exothermic composition may further optionally contain other components that can be incorporated into an exothermic composition. Examples of such components include, but are not limited to, surfactants, hydrogen-generation inhibitors, thickening agents, excipients, metal ion sequestrants, and fragrances. Examples of fragrances are shown below. In the present invention, however, the adhesive layer contains an active ingredient, such as a fragrance; thus, a fragrance may not be incorporated into the exothermic composition. If the exothermic composition contains a fragrance, this fragrance preferably does not hinder the effects of the fragrance contained as an active ingredient in the adhesive layer.

In the exothermic composition, the total amount of the oxidation accelerator, oxidizable metal powder, and water is not limited as long as the desired effects are obtained, and may be arbitrarily set so that the exothermic temperature of the exothermic composition becomes a temperature suitable for a heating tool (e.g., about 32 to 85°C, as measured based on JIS S4100).

In one embodiment of the exothermic composition used for the heating tool of the present invention, for example, the exothermic composition contains the oxidation accelerator in an amount of 1 to 30 wt%, the oxidizable metal powder in an amount of 20 to 80 wt%, and water in an amount of 5 to 50 wt%.

In another embodiment of the exothermic composition used for the heating tool of the present invention, for example, the exothermic composition contains the oxidation accelerator in an amount of 1 to 30 wt%, the oxidizable metal powder in an amount of 20 to 80 wt%, the water-soluble salt in an amount of 0.1 to 10 wt%, and water in an amount of 5 to 50 wt%.

In another embodiment of the exothermic composition used for the heating tool of the present invention, for example, the exothermic composition contains the oxidation accelerator in an amount of 1 to 30 wt%, the oxidizable metal powder in an amount of 20 to 80 wt%, the water-retaining agent in an amount of 1 to 20 wt%, and water in an amount of 5 to 50 wt%.

In another embodiment of the exothermic composition used for the heating tool of the present invention, for example, the exothermic composition contains the oxidation accelerator in an amount of 1 to 30 wt%, the oxidizable metal powder in an amount of 20 to 80 wt%, the water-soluble salt in an amount of 0.1 to 10 wt%, the water-retaining agent in an amount of 1 to 20 wt%, and water in an amount of 5 to 50 wt%. Further, for example, the exothermic composition contains the oxidation accelerator in an amount of 5 to 23 wt%, the oxidizable metal powder in an amount of 30 to 60 wt%, the water-soluble salt in an amount of 1 to 5 wt%, the water-retaining agent in an amount of 5 to 10 wt%, and water in an amount of 15 to 35 wt%.

Additionally, in one embodiment of the exothermic composition used for the heating tool of the present invention, for example, the composition contains carbon black, iron powder, and water. In another embodiment of the exothermic composition used for the heating tool of the present invention, for example, the composition contains carbon black, iron powder, sodium chloride, and water. In another embodiment of the exothermic composition used for the heating tool of the present invention, for example, the composition contains carbon black, iron powder, a water-absorbing resin and/or vermiculite, and water. In another embodiment of the exothermic composition used for the heating tool of the present invention, for example, the composition contains carbon black, iron powder, sodium chloride, a water-absorbing resin and/or vermiculite, and water.

The exothermic composition is prepared by mixing the components described above. The exothermic composition may be prepared in the presence of oxygen, or may be prepared in vacuum or in an inert gas atmosphere. The preparation may be performed in accordance with hitherto-known procedures. In this manner, it is possible to obtain an exothermic composition that generates heat in the presence of air.

### Air-Permeable Container Bag for Housing Exothermic Composition

The exothermic composition is housed in an air-permeable container bag for housing the exothermic composition (hereinafter, sometimes referred to as "container bag for the exothermic composition"). The container bag for housing the exothermic composition is not limited as long as the bag is capable of housing the exothermic composition and has air-permeability. It is possible to use hitherto-known container bags. For example, air-permeable bags or the like used for hitherto-known disposable body warmers may be used as the container bag, for example, from the standpoint that such air-permeable bags can prevent leakage of the exothermic composition, have durability against heat generated by the exothermic composition, and can improve the ease of use of the heating tool.

In a more specific non-limiting example, the container bag has a laminated structure in which an air-permeable resin film is laminated to an air-permeable woven fabric or nonwoven fabric. In this case, the air-permeable resin film is disposed inside the container bag for the exothermic composition, and the air-permeable woven fabric or nonwoven fabric is disposed outside the container bag for the exothermic composition.

The resin used for the air-permeable resin film is not particularly limited. Preferable examples thereof include thermoplastic resins. Examples of thermoplastic resins include polyethylene, polypropylene, polyester, polyamide, polyurethane, polystyrene, polyvinyl alcohol, polyvinyl chloride, polyvinylidene chloride, polycarbonate, ethylene-vinyl acetate copolymers, and the like. Preferable examples of thermoplastic resins include polyethylene, polypropylene, ethylene-vinyl acetate copolymers, and the like, from the standpoint of usage of the heating tool as being attached to the body. These may be used singly, or in a combination of two or more.

In terms of the air-permeable resin film used in the present invention, pores for ensuring the air permeability are provided at least partially with the resin film formed of the resin described above. The pores are not limited as long as they allow air to move in and out of the container bag for the exothermic composition, and as long as they are of sufficient size to prevent the exothermic composition from leaking to the outside of the container bag. Further, the sensible temperature at the time of use of the heating tool can be affected by the air permeability of the container bag for the exothermic composition; therefore, the size, shape, and number of pores may be arbitrarily determined in consideration of the sensible temperature at the time of use of the heating tool. Means for forming pores in the resin film are hitherto known, and a known procedure can be followed.

Examples of the fiber materials of the air-permeable woven fabric or nonwoven fabric include synthetic fibers such as nylon, vinylon, polyester, rayon, acrylic, polyethylene, polypropylene, acetate, polyvinyl chloride, and polybutylene terephthalate, natural fibers such as cotton, hemp, silk, and paper, mixed fibers of synthetic fibers and natural fibers, and the like. From the perspective of sense of use, examples of the fiber materials include nylon, polyester, polypropylene, and the like, and more preferably nylon and polyester. These may be used singly, or in a combination of two or more. The woven fabric or nonwoven fabric is not limited as long as the fabric allows air to move in and out of the container bag for the exothermic composition, and can prevent the exothermic composition from leaking to the outside of the container bag. The weight per area of the fabric is preferably, for example, 20 to 70 g/m².

The laminate of the air-permeable resin film to air-permeable woven fabric or nonwoven fabric is not limited as long as the obtained laminated body is strong enough to serve as the container bag for the exothermic composition, and exhibits air permeability. The laminate may be performed by, for example, a lamination method. Examples of the lamination method include methods of laminating by thermal bond, and methods of laminating using adhesives such as hot-melt adhesives, acrylic adhesives, or urethane adhesives. The laminate may be performed partially or entirely to the surface of the container bag for the exothermic composition, as long as the desired effects are obtained.

It is also possible to use commercially available products as the container bag for the exothermic composition.

The size and shape of the container bag for the exothermic composition are also not limited as long as the desired effects are obtained, and may be arbitrarily determined depending on the purpose of use.

### Active Ingredient-Containing Adhesive Layer

In the present invention, the adhesive layer contains an active ingredient. The active ingredient is not limited as long as the effects of the present invention are obtained, and is arbitrarily used according to the purpose, in consideration of hitherto-known useful effects of each component.

Without limiting the present invention, examples of the active ingredient include fragrances. The fragrances may be, for example, natural fragrances, such as an essential oil, which may be used singly or in combination; synthetic single fragrances, which may be used singly or in combination; and compound fragrances obtained by freely combining natural fragrances and synthetic fragrances.

Without limiting the present invention, examples of the natural fragrances (essential oils) include vanilla, lavender, chamomile, rosemary, sage, citronella, ginger, ylang-ylang, eucalyptus, mint, rose, lily, lilac, jasmine, cardamom, lemon grass, yuzu, orange, lemon, lime, grapefruit, neroli, cedar wood, sandalwood, anise, caraway, amber, musk, civet, castoreum, basil, lavandin, peppermint, cypress, juniper, geranium, thyme, bergamot, and the like. Examples of the synthetic single fragrances include acetophenone, aldehyde C₆-C₁₆, allyl caproate, amylcinnamic aldehyde, amyl salicylate, benzaldehyde, benzyl acetate, benzyl alcohol, borneol, camphor, cinnamic alcohol, citral, citronellal, citronellol, coumarin, damascone, dehydrolinalool, dihydromyrcenol, diphenyl oxide, ethyl-2-methyl butyrate, ethyl butyrate, eugenol, geraniol, geranyl acetate, phenylethyl alcohol, hedione, hexanol, cis-3-hexanol, α-hexyl cinnamic aldehyde, isoamyl acetate, lilial, limonene, linalool, linalyl acetate, 1-menthol, methyl benzoate, methyl ionone, methyl salicylate, nerol, α-pinene, β-pinene, rose oxide, terpineol, γ-nonalactone, γ-undecalactone, vanillin, and the like.

As described above, the useful effects of each component are hitherto known. For example, sage and the like serve as warm-sensation components; basil, juniper, and the like serve as anti-fatigue components; lavandin, lavender, grapefruit, rosemary, peppermint, chamomile, lemon, eucalyptus, jasmine, rose, and the like serve as analgesic components; lemon, eucalyptus, basil, and the like serve as inflammation-inhibiting components; grapefruit, rosemary, lemon, ginger, and the like serve as blood circulation-promoting components; lavender, chamomile, ylang-ylang, sage, cypress, sandalwood, jasmine, juniper, geranium, neroli, basil, rose, bergamot, and the like serve as relaxation components; 1-menthol, camphor, peppermint, chamomile, cypress, juniper, geranium, bergamot, lavender, lemon grass, rosemary, and the like serve as cool sensation (cooling, refreshment) components; eucalyptus, pyrethroid, paramenthane, and the like serve as repellent components; cedar wood and the like serve as sedative components; basil, lavandin, and the like serve as muscle-rigidity-suppressing components; cypress and the like serve as tonics for circulatory organ systems; juniper, thyme, and the like serve as detoxification components; juniper, basil, bergamot, and the like serve as stimulus (exaltation) components; and eucalyptus serves as head-clearing components.

Examples of warm-sensation components include capsaicin, caffeine, tocopherol nicotinate, nonylic acid vanillyl amide, nicotinic acid benzyl ester, vanillyl butyl ether, vanillyl ethyl ether, gingerol, vanillyl propyl ether, vanillyl pentel ether, vanillyl hexyl ether, vanillyl butyl ether acetate, capsicum extract, and the like. Examples of moisturizing components include ceramide, hyaluronic acid, squalane, collagen, Lipidure (registered trademark), and the like.

Examples of other active ingredients include vitamin A, retinol, palmitic acid, tetrahexyldecanoic acid, ascorbic acid, tocopherol, dl-α-tocopherol, acetic acid dl-α-tocopherol, L-menthol, camphor, sulfur, pyridoxine chloride, sorbitol, maltitol, hyaluronic acid, cork tree bark extract, glycyrrhiza extract, aloe extract, seaweed extract, carrot extract, mulberry extract, components contained in hitherto-known poultices, antipyretic components, components effective in treating stiffness in shoulder, and the like. The active ingredients are not limited, and any component may be used according to the purpose.

These active ingredients may be used singly, or in a combination of two or more.

As described above, examples of various useful effects include warm-sensation, anti-fatigue, pain relief, inflammation inhibition, blood circulation promotion, relaxation, cool sensation (cooling, refreshment), repellent, sedation, moisturization, skin-whitening, anti-wrinkle, aging prevention, antibacterial, anti-oxidation, slimming, cleansing, circulatory organ system invigoration, perfuming, nervous edema inhibition, muscle rigidity suppression, detoxification (including discharge of unnecessary wastes and water), skin invigoration, immune function improvement, stimulus (exaltation), head-clearing, and activation. Thus, the heating tool of the present invention further achieves an effect (function) associated with the characteristics of the active ingredient used. For example, when an analgesic component is contained as the active ingredient, the heating tool of the present invention is considered to be a tool that exerts an analgesic effect (an analgesic tool). When an active ingredient having an inflammation inhibitory effect, blood circulation promotion effect, or the like is used, the heating tool of the present invention is respectively considered to be a tool that exerts an inflammation inhibitory effect (an inflammation inhibition tool), a tool that exerts a blood-circulation promotion effect (a blood-circulation promotion tool), or the like.

To more effectively exert a useful effect of an active ingredient with heat generated in the heating tool, the active ingredient is preferably a component that can vaporize at a temperature at which the exothermic composition generates heat in the presence of air (e.g., about 32 to 85°C). The active ingredient may be liquid, solid, or the like.

The amount of the active ingredient in the adhesive layer is also not limited as long as the desired effects are obtained. The proportion of the active ingredient in the adhesive layer is, for example, 0.00001 to 85 wt%, preferably 0.0001 to 10 wt%, and more preferably 0.0001 to 5 wt%.

Without limiting the present invention, from among the oxidation accelerators stated above, for example, carbon black can relatively suppress the absorption of the active ingredient. However, even if carbon black is used, the active ingredient is preferably, for example, an oil-soluble component, from the standpoint of more efficiently obtaining a useful effect attributable to the active ingredient. of active ingredients, oil-soluble active ingredients are likely to be absorbed on carbon black. Thus, when carbon black is used with an oil-soluble active ingredient, an absorption problem of the active ingredient is likely to occur. However, even with such a combination, it is possible in the present invention to significantly suppress a reduction of the active ingredient contained in the adhesive layer. Oil-soluble active ingredients are components known to a person skilled in the art, are insoluble or poorly soluble in water at room temperature (25°C), and are easily dissolved in an organic solvent or an oil and fat at room temperature (25°C). While not limiting the present invention, examples of oil-soluble active ingredients include various components, such as the fragrances mentioned above, capsaicin, caffeine, tocopherol nicotinate, nonylic acid vanillyl amide, nicotinic acid benzyl ester, vanillyl butyl ether, vanillyl ethyl ether, gingerol, vanillyl propyl ether, vanillyl pentel ether, vanillyl hexyl ether, vanillyl butyl ether acetate, and the like.

The adhesive layer may further contain a base. The base is not limited as long as it is harmless when in contact with the skin, and as long as the adhesiveness is achieved even when combined with an active ingredient to an extent that the adhesive layer is allowed to be attached to the skin. Such a base is not limited as long as the desired effects are obtained, and examples thereof include those that are hitherto used as an adhesive component in, for example, patches for application to the skin. Thus, examples of the base include hitherto-known adhesive components.

Examples of the base include rubber-based adhesives, such as styrene-butadiene-styrene-based copolymers (SBS), styrene-isoprene-styrene-based copolymers (SIS), and hydrogenated copolymers thereof (i.e., styrene-ethylene-butylene-styrene copolymers (SEBS) and styrene-ethylene-propylene-styrene copolymers (SEPS)); acrylic-based adhesives, such as methyl acrylate (MA), ethyl acrylate (EA), 2-ethylhexyl acrylate (HA), and butyl acrylate (BA); silicone-based adhesives, such as addition cure type or peroxide cure type silicone-based adhesives; urethane-based adhesives, such as ether type or ester type urethane-based adhesives; and the like. The base is preferably, for example, rubber-based adhesives, and more preferably styrene-butadiene-styrene-based copolymers (SBS), styrene-isoprene-styrene-based copolymers (SIS), and hydrogenated copolymers thereof (i.e., styrene-ethylene-butylene-styrene copolymers (SEBS) and styrene-ethylene-propylene-styrene copolymers (SEPS)). These may be used singly, or in a combination.

The amount of the base is not limited as long as the desired effects are obtained. The proportion of the base in the adhesive layer is 10 to 99 wt%, more preferably 30 to 98 wt%, and more preferably 50 to 97 wt%.

As long as the desired effects are obtained, the adhesive layer may further optionally contain an arbitrary component, such as an oil, an antiseptic, and a colorant, in addition to the components described above.

In the present invention, the adhesive layer is laminated to the outside of the container bag for the exothermic composition via a vapor deposition layer described later. The lamination of the adhesive layer is not limited as long as the adhesive layer is laminated to the outside of the container bag via a vapor deposition layer described later. For example, the lamination may be conducted by applying a mixture obtained by mixing the active ingredient described above, the base described above, and optionally, the arbitrary component described above to a nonwoven fabric, a resin film, a woven fabric, a paper, or the like provided on a vapor deposition layer described later. This is easily performed by a person skilled in the art. It is also possible to apply a mixture obtained by mixing the active ingredient described above, the base described above, and optionally, the arbitrary component described above to a nonwoven fabric or the like, and further applying the obtained adhesive layer to a vapor deposition layer. This is easily performed by a person skilled in the art. The procedure shown in the Examples below serves as an example.

Furthermore, a film, such as silicon-treated polyethylene terephthalate or polypropylene, or a paper may be attached to the adhesive layer surface to desirably use the heating tool while, for example, preventing the adhesive layer surface from drying. This film is removed at the time of use of the heating tool.

The thickness, size, shape, and the like of the adhesive layer to be laminated are not limited as long as the effects of the present invention are obtained, and are arbitrarily determined according to the purpose of use, such as application site of the heating tool comprising the adhesive layer. To efficiently obtain the effects of the present invention, the thickness of the adhesive layer is, for example, 1 to 3000 µm, and more preferably 10 to 300 µm. The size of adhesive layer is arbitrarily determined depending on the application site of the body, such as eyes, shoulders, waist, back, arms, legs, and soles. The shape is also not limited as long as the effects of the present invention are obtained, and arbitrarily determined depending on, for example, the application site so that the heating tool is easily used. The adhesive layer may be laminated entirely or partially to the outside of the container bag for the exothermic composition. The portion where the adhesive layer is laminated is not limited as long as the effects of the present invention are obtained. It is also possible that the adhesive layer be laminated entirely or partially to the vapor deposition layer. As long as the adhesive layer is partially laminated to the vapor deposition layer, a portion or portions of the adhesive layer may be directly laminated to the container bag, and as long as the effects of the present invention are obtained, the portion to which the adhesive layer is laminated is also not limited.

The heating tool of the present invention is not limited as long as the effects of the present invention are obtained. For example, the heating tool of the present invention may be directly attached to the skin via the adhesive layer, or attached through a garment or the like via the adhesive layer. To more effectively provide a useful effect attributable to an active ingredient with the application site, the heating tool of the present invention is directly attached to the skin via the adhesive layer.

### Vapor Deposition Layer

The heating tool of the present invention is provided with a vapor deposition layer, and the vapor deposition layer is disposed between the adhesive layer and the container bag for the exothermic composition.

The vapor deposition layer is not limited as long as the effects of the present invention are obtained. Examples include a vapor deposition layer containing a metal, such as aluminum, chromium, zinc, gold, silver, platinum, and nickel; an oxide, such as silicon dioxide, titanium oxide, aluminum oxide, and zirconium oxide; a fluoride, such as magnesium fluoride; a nitride, such as titanium nitride; and a carbide, such as titanium carbide. Preferable examples include a vapor deposition layer containing a metal, such as aluminum, chromium, zinc, gold, silver, platinum, and nickel; and an oxide, such as silicon dioxide, titanium oxide, aluminum oxide, and zirconium oxide. These may be used singly, or in a combination.

In the present invention, the vapor deposition layer is laminated to the outside of the container bag for the exothermic composition. The thickness, size, shape, and the like of the vapor deposition layer are not limited as long as the effects of the present invention are obtained, and are arbitrarily determined according to the purpose of use, such as application site of the heating tool comprising the vapor deposition layer. To efficiently obtain the effects of the present invention, the thickness of the vapor deposition layer is, for example, 1 to 1000 nm, and more preferably 5 to 100 µm. The size of the vapor deposition layer is also arbitrarily determined depending on the application site. The shape is also not limited as long as the effects of the present invention are obtained, and arbitrarily determined depending on, for example, the application site so that the heating tool is easily used. The vapor deposition layer may be laminated entirely or partially to the outside of the container bag for the exothermic composition. The portion where the vapor deposition layer is laminated is not limited as long as the adhesive layer is laminated to the container bag via the vapor deposition layer.

The vapor deposition of the components above may be performed according to hitherto-known vapor deposition methods, such as physical vapor deposition (PVD) and chemical vapor deposition (CVD). The vapor deposition layer may be produced by vapor deposition of, for example, the metal, oxide, fluoride, nitride, or carbide mentioned above on any film such as polyethylene terephthalate, nylon, or polypropylene.

As long as the vapor deposition layer is disposed on the outside the container bag in the above manner, the method for disposing the vapor deposition layer between the adhesive layer and the container bag is not limited. Without limiting the present invention, the procedure shown in the Examples below serves as an example. In this manner, it is possible to obtain a heating tool in which the vapor deposition layer is disposed between the container bag and the adhesive layer.

The heating tool of the present invention, which is arranged in the above manner, can exert an excellent useful effect attributable to an active ingredient at the time of use, even after long-term storage. In particular, according to the heating tool of the present invention, a reduction of the active ingredient contained in the adhesive layer is significantly suppressed while using the heat-generating mechanism; this makes it possible to more effectively exert the useful effect attributable to the active ingredient. In particular, for example, carbon black from among the oxidation accelerators mentioned above can suppress the absorption of a component relatively satisfactorily while using the heat-generating mechanism. However, according to the present invention, the absorption of an active ingredient can be further suppressed even with the use of the heat-generating mechanism in which such carbon black is used, making it possible to more effectively exert the useful effect attributable to the active ingredient.

Therefore, the present invention also provides a method for suppressing a reduction of an active ingredient in the heating tool comprising the active ingredient-containing adhesive layer by disposing the vapor deposition layer between the active ingredient-containing adhesive layer and the container bag housing the exothermic composition containing the oxidation accelerator, the oxidizable metal powder, and water; and a method for more effectively exerting an useful effect attributable to an active ingredient. The oxidation accelerator, oxidizable metal powder, water, exothermic composition, container bag, adhesive layer, vapor deposition layer, heating tool, and other components, the amounts of these components, the production methods, the application methods, and the like used in these methods are as defined above.

The thus-produced heating tool is, in general, further packaged in an air-impermeable outer bag that does not allow permeation of oxygen, so as to be supplied or stored while maintaining an airtight state. In the heating tool of the present invention, the exothermic composition generates heat when in contact with oxygen to thus achieve sufficient exothermic effects, as in general-purpose body warmers. Further, the heating tool of the present invention more effectively provides, together with the exothermic effect, the useful effect attributable to the active ingredient contained in the adhesive layer at a site to which the heating tool is applied. Therefore, it is important to store the heating tool in such a manner that the heating tool would not come into contact with oxygen during storage to prevent the heating tool from generating heat before being used. At the time of use of the heating tool of the present invention, the outer bag is opened to remove the heating tool from the outer bag, so that the exothermic composition is brought into contact with oxygen to generate heat. The outer bag used herein is not particularly limited as long as it is an air-impermeable bag that does not allow permeation of oxygen.

### Examples

The following describes the present invention with reference to Examples; however, the present invention is not limited to the following Examples.

### 1. Example: Production of Heating Tools 1 and 2

Heating tools 1 and 2 were produced in accordance with the following procedures.

### 1-1. Production of Heating Tool 1

A heating tool 1 was produced in accordance with the following procedures.

### - Vapor Deposition Layer

An alumina (Al₂O₃) vapor deposition layer having a thickness of 100 nm or less formed by physical vapor deposition was formed on a polyethylene terephthalate resin film (hereinafter referred to as "a PET film"); then, a polyethylene film (hereinafter referred to as "a PE film") was applied to the vapor deposition layer side by dry lamination (product name: GL-AE, produced by Toppan Printing Co., Ltd.); and a polyester nonwoven fabric is applied to the PET film side, which is opposite to the vapor deposition layer side, by dry lamination.

### - Active Ingredient-Containing Adhesive Layer

Vanillyl butyl ether (produced by Takasago International Corporation), an oil, a colorant, a hot melt adhesive, and the like were mixed under heating at 140°C, and the resulting mixture was applied in an amount of 100 g/m² to the polyester nonwoven fabric dry laminated in the "Vapor Deposition Layer" section above, followed by cooling to thus form an adhesive layer on the polyester nonwoven fabric. Subsequently, a silicon-treated PET film (release film) was bonded at the adhesive layer side. The vanillyl butyl ether was used as an active ingredient.

### - Exothermic Composition Housed in Container Bag

The following components were used in an exothermic composition.
- Iron powder (produced by Dowa IP Creation Co., Ltd., product name: DKP)
- Carbon black (produced by Cabot Japan, product name: Show Black IP200)
- Water
- Vermiculite
- Water-absorbent resin (acrylic acid polymer partially crosslinked product)
- Salt

The above exothermic composition components were mixed to obtain an exothermic composition. Here, the proportions of the iron powder, the carbon black, the water, the vermiculite, the water-absorbent resin, and the salt in the exothermic composition were respectively 55 wt%, 8 wt%, 28.5 wt%, 5 wt%, 2 wt%, and 1.5 wt%.

Subsequently, the circumference of the PE film formed on the vapor deposition layer on the vapor deposition layer side was bonded to the circumference of a nonwoven fabric/polyethylene breathable film (product name: Breathron, produced by Nitto Denko Corporation) by thermal bonding to form a sealed bag, while the obtained exothermic composition was filled therein in an amount per unit area of 0.25 g/cm², to thus obtain an exothermic composition housed in a container bag. The moisture permeability of this container bag was 330 g/m²•day, as measured by the Lyssy method.

After the vapor deposition layer and the adhesive layer were laminated to the container bag as described above, the resulting product was quickly packaged in an air-impermeable outer bag. Fig. 1 shows a model drawing of the obtained heating tool.

### 1-2. Production of Heating Tool 2

A heating tool 2 was produced as in the heating tool 1, except that an aluminum vapor deposition PET film treated with silicon was used as a release film, in place of the silicon-treated PET film, and the resulting product was quickly packaged in an air-impermeable outer bag.

### 2. Comparative Example: Production of Comparative Heating Tool and Patch

### 2-1. Production of Comparative Heating Tool

A comparative heating tool was produced as in the heating tool 1, except that a styrene-ethylene-butylene-styrene copolymer (SEBS) was laminated by an extrusion method between a polyolefin-based film and the polyester nonwoven fabric, in place of the laminated body obtained in the "Vapor Deposition Layer" section above. The resulting product was then quickly packaged in an air-impermeable outer bag.

### 2-2. Production of Patch

An adhesive layer and a release film, as described in the "Active Ingredient-Containing Adhesive Layer" section above, were laminated to the polyester nonwoven fabric to produce a patch, which was quickly packaged in an air-impermeable outer bag.

### 3. Test Example

The amounts of active ingredient (vanillyl butyl ether) contained in the adhesive layer used in the heating tools 1 and 2, the comparative heating tool, and the patch were determined by chromatography mass spectrometry in accordance with the following procedures.

Specifically, the heating tools 1 and 2, the comparative heating tool, and the patch were packaged in a respective outer bag as described above, and stored for a certain period of time. The time after completion of the storage for a certain period of time was considered as the start time of the test, and the heating tools 1 and 2, the comparative heating tool, and the patch were further stored for one month at 50°C with a relative humidity of 50%. This storage was equivalent to 1.5-year storage at room temperature (25°C). After the storage, and after removal from the outer bag, the release film provided in the "Active Ingredient-Containing Adhesive Layer" section above was peeled off, and a certain amount of only adhesive layer and polyester nonwoven fabric (hereinafter referred to as a sample) was collected, to which chloroform was added, so that the sample weight (g):chloroform (ml) = about 1:50. Then, ultrasonic extraction was carried out for 10 minutes, and the chloroform layer was used as a test solution. This test solution was infused into the chromatography mass spectrometer to quantify the vanillyl butyl ether in the sample.

In the present invention, in the "Active Ingredient-Containing Adhesive Layer" section above, the adhesive layer was partially entwined with the fiber of the nonwoven fabric. In view of the fact that a nonwoven fabric does not dissolve in chloroform, the sample after extraction was washed with chloroform and dried at 80°C overnight, and the weight of the nonwoven fabric was measured. Considering the difference between the sample weight before extraction and the sample weight after drying (nonwoven fabric) to be the weight of the adhesive layer, the amount (wt%) of vanillyl butyl ether in the adhesive layer was calculated.

Subsequently, the calculated amount of the vanillyl butyl ether after storage was divided by the amount of vanillyl butyl ether in the adhesive layer at the time that the test was started (0.28 wt%) to calculate the residual rate of vanillyl butyl ether in the adhesive layer after storage.

The following are operating conditions for a chromatography mass spectrometer:
Model: 6890/5973 (produced by Hewlett-Packard Co., Ltd.)
Column: DB-WAX (produced by Agilent Technologies), ϕ 0.25 mm x 30 m, Film thickness: 0.25 µm
Inlet type: Split (1:30)
Temperature: Sample inlet, 220°C

Column, 60°C (maintained for 1 minute) → heated (10°C/min) → 250°C
Gas flow rate: helium (carrier gas) 1 ml/min
Ion source temperature: 230°C
Ionization method: EI
Set mass number: m/z 210, 137
Table 1 shows the results.

**Table 1**

| | Residual ratio (%) of vanillyl butyl ether in the adhesive layer | | | |
|---|---|---|---|---|
| | Heating tool 1 | Heating tool 2 | Comparative heating tool | Patch |
| One-month storage at 50°C with a relative humidity of 50% | 89.3% | 96.4% | 0.75% | 100% |

After being packaged in an outer bag and from the start time of the test, the heating tool 1 was further stored for 3 months or 6 months at 40°C with a relative humidity of 75%, and the residual ratio of vanillyl butyl ether in the adhesive layer was calculated in the same manner as above. As a result, the residual ratio after 3-month storage was 82.1%, and after 6-month storage was 67.9%.

As is clear from the results of Table 1 above, when the vapor deposition layer was disposed between the container bag housing an exothermic composition and the active ingredient-containing adhesive layer (the heating tools 1 and 2), a reduction of the active ingredient in the adhesive layer was significantly suppressed. In contrast, the comparative heating tool, which did not have the vapor deposition layer, resulted in a great reduction of the active ingredient in the adhesive layer. This reveals that the lamination of the vapor deposition layer significantly suppresses a reduction of the active ingredient contained in the adhesive layer, making it possible to very effectively achieve the useful effect attributable to the active ingredient.

Although the vapor deposition layer was not used in the patch, similar to the comparative heating tool, the patch did not show a significant reduction of the active ingredient, as seen in the comparative heating tool. The main difference between the comparative heating tool and the patch is that the former is provided with an exothermic composition, while the latter is not provided with an exothermic composition. These results reveal that a significant reduction of the active ingredient in the adhesive layer is a notably specific problem only in a heating tool that uses an exothermic composition in combination with an active ingredient in the adhesive layer, and patches that are not provided with an exothermic composition originally do not suffer from this problem.

Further, although not shown in the results, when a silica (silicon dioxide)-deposition layer was provided in the test above in place of alumina vapor deposition, a reduction of the active ingredient in the adhesive layer was also significantly suppressed, which indicates that the useful effect attributable to the active ingredient was exerted effectively.

Further, in this test example, carbon black, which does not easily absorb active ingredients or other substances, was used as the oxidation accelerator. That is, under the conditions of this test example, a reduction of the active ingredient would, originally, not easily occur. Even under such conditions, it was possible to significantly suppress a reduction of the active ingredient when a vapor deposition layer was further provided. Further, in this test example, in particular, an oil-soluble component, which is easily absorbed on carbon black, was used as an active ingredient; however, the provision of the deposition layer significantly suppressed a reduction of the active ingredient in the adhesive layer. It is a very surprising effect that a reduction of the active ingredient can be significantly suppressed even under these conditions.

Further, the heating tool used herein comprises the exothermic composition, and is thus capable of exerting a sufficient exothermic effect.

## Claims

1. A heating tool comprising:
an exothermic composition containing an oxidation accelerator, an oxidizable metal powder, and water;
an active ingredient-containing adhesive layer; and
a vapor deposition layer, the exothermic composition being housed in an air-permeable container bag, the vapor deposition layer being disposed between the container bag and the adhesive layer.

2. The heating tool according to claim 1, for direct application to the skin via the adhesive layer.

3. The heating tool according to claim 1 or 2, wherein the oxidation accelerator is carbon black.

4. The heating tool according to any one of claims 1 to 3, wherein the exothermic composition further contains a water-soluble salt and/or a water-retaining agent.

5. The heating tool according to any one of claims 1 to 4, wherein the exothermic composition contains the oxidation accelerator in an amount of 1 to 30 wt%.

6. The heating tool according to any one of claims 1 to 5, wherein the adhesive layer contains the active ingredient in an amount of 0.00001 to 85 wt%.

7. A method for suppressing a reduction of an active ingredient in a heating tool comprising an active ingredient-containing adhesive layer, the method comprising disposing a vapor deposition layer between the active ingredient-containing adhesive layer and a container bag housing an exothermic composition containing an oxidation accelerator, an oxidizable metal powder, and water.
